# EUROPÄISCHE PATENTSCHRIFT

(11) **EP 3 648 712 B1**
(45) Veröffentlichungstag und Bekanntmachung des Hinweises auf die Patenterteilung: **11.11.2020**
(21) Anmeldenummer: 18750136.6
(22) Anmeldetag: 31.07.2018
(51) Int. Cl.: A61F 5/30, A61F 2/60, A61F 2/64, A61F 5/01

(54) **GELENKORTHESE MIT BEWEGLICHER PELOTTE**
JOINT ORTHOSIS WITH MOVABLE PAD
ORTHÈSE ARTICULAIRE DOTÉE D'UNE PELOTE MOBILE

(30) Priorität: 01.08.2017 DE 102017213300
(43) Veröffentlichungstag der Anmeldung: 13.05.2020
(73) Patentinhaber: Bauerfeind AG, 07937 Zeulenroda-Triebes (DE)
(72) Erfinder: HESS, Heinrich, 66271 Kleinblittersdorf (DE); BAUERFEIND, Hans B., 07937 Zeulenroda-Triebes (DE)
(74) Vertreter: Schwahn, Hartmut
(86) Internationale Anmeldenummer: PCT/EP2018/070669
(87) Internationale Veröffentlichungsnummer: WO 2019/025403

(56) Entgegenhaltungen:
- DE-A1-102012 218 804
- US-A1- 2012 289 878
- US-A1- 2013 296 757

## Beschreibung

Die vorliegende Offenbarung betrifft eine aktive Gelenkorthese, besonders eine Orthese des Hüftgelenks, mit erweiterter therapeutischer Funktion durch eine Gelenkschiene mit aktiv bewegter Pelotte.

Gelenkübergreifende Gelenkorthesen zur Stützung oder Führung einer Gelenkbewegung eines Körpergelenks sind bekannt. Diese weisen üblicherweise eine Fassung oberhalb des zu stützenden Körpergelenks und eine Fassung unterhalb davon auf. Diese Fassungen sind über mindestens eine Gelenkschiene verbunden. Die Gelenkachse der Gelenkschiene ist dabei im Bereich des Körpergelenks positioniert. Durch die gelenkige Verkopplung von unterer und oberer Fassung wird die Gelenkorthese zusammen mit dem Gelenk bei dessen Beugung oder Streckung geführt und kann so die Gelenkbewegung unterstützen oder führen. Gelenkorthesen für das Hüftgelenk, sollen beispielsweise für die Behandlung der Hüftarthrose oder Coxarthrose geeignet sein und sollen das Hüftgelenk entlasten. Bei einer Hüftorthese wird die obere Fassung (Hüftteil) zunächst durch einen Beckengurt gebildet, der den Beckenkamm fest umgreift. Mit dem Beckengurt verbunden ist der obere Abschnitt oder Arm der Gelenkschiene. Die untere Fassung umgreift mit Gurten den Oberschenkel. Die Oberschenkelfassung ist mit dem unteren Abschnitt oder Arm der Gelenkschiene fest verbunden.

US2013296757 als nächster Stand der Technik beschreibt eine Orthese umfassend: eine Manschette, die zum Umgreifen eines Gliedes unterhalb eines Gelenks konfiguriert ist und die mit einem Widerlager oberhalb des Gelenks gekoppelt ist; ein Spannband welches kraftschlüssig mit dem Widerlager und der Manschette verbunden ist; wobei die Manschette einen druckauslösenden Abschnitt (z.B. ein Druckpolster) aufweist, der mit dem Spannband gekoppelt ist, und das Spannband so konfiguriert ist, dass es durch die Bewegung des Gliedmaßengelenks gedehnt wird und dadurch über den druckauslösenden Abschnitt auf einen darunter liegenden Weichteilbereich der Extremität Druck ausgeübt wird. DE102012218804 offenbart eine Orthese bei der durch eine entlang der Gelenkachse translatorisch bewegbar angeordnete Korrekturpelotte (= Druckglied) ein definiertes Valgus- oder Varusmoment in Abhängigkeit von einer jeweiligen Schwenkstellung der beiden Schienenteile zueinander, d. h., in Abhängigkeit von einer jeweiligen Flexion des mit der Orthese versorgten Gelenks, auf das Gelenk ausgeübt wird.

Nachteilig bei bekannten Gelenkorthesen ist, dass durch die für die entsprechende Stützwirkung erforderliche mechanisch stabile Konstruktion Vergurtungen und Bandagenelemente notwendig werden, worunter der Tragekomfort der Orthese im Gebrauch leidet. Die Akzeptanz (Compliance) solcher Orthesen beim Patienten ist daher nicht immer gut, vor allem auch deswegen, weil durch die zu behandelnde bestehende Arthrose erhebliche Schmerzen bei der Gelenkbewegung auftreten können. Diese Schmerzen entstehen insbesondere deswegen, weil infolge der arthrosebedingten chronischen Entzündung die Gelenkweichteile (Kapsel etc.) kontrakt, d.h. geschrumpft, sind, die Beweglichkeit immer mehr eingeschränkt wird und zwangsläufig Bewegungen in dem Gelenk schmerzhaft werden. Eine ganz wesentliche Therapie zur Erweiterung des Bewegungsspielraums durch Dehnung der kontrakten Gelenkweichteile ist zum Beispiel die von Physiotherapeuten ausgeübte nach distal gerichtete Extensionsbehandlung durch Zug am Bein. Dieser schmerzlindernde Zug könnte auch durch eine Orthese nachempfunden werden. Gelänge es, das Tragen der stützenden Orthese mit einer gleichzeitigen Schmerzlinderung zu verbinden, könnte der Tragekomfort, die Compliance und damit letztlich der Therapieerfolg verbessert werden. Solche Probleme bestehen besonders bei Hüftgelenken, aber auch anderen arthrotisch abgenutzten Körpergelenken wie Kniegelenken.

Es ist daher wünschenswert, derart verbesserte Gelenksorthesen bereitzustellen, die neben einer ausreichenden stützenden oder gelenkführenden orthetischen Funktion, zusätzlich eine schmerzlindernde Wirkung auf die betroffenen Gelenke und Körperabschnitte besitzen. Das der vorliegenden Erfindung zugrunde liegende technische Problem bestand daher in der Bereitstellung einer verbesserten Gelenkorthese, deren Tragekomfort und Compliance verbessert sind.

Das technische Problem wird gelöst durch eine neuartige Gelenkschienenkonstruktion, welche an einem der beiden Gelenkarme der Gelenkschiene eine aktiv bewegbare Pelotte zur Massage eines Körperabschnitts aufweist, wobei die Pelottenbewegung im angelegten Zustand und bei Gebrauch der Orthese aktiv über die Verschwenkung der beiden Gelenkarme der Gelenkschiene zueinander, das heißt bei Beugung oder Streckung des Körpergelenks, vermittelt wird.

Dazu stellt die Erfindung eine Orthesengelenkschiene nach Anspruch 1 für eine Gelenkorthese bereit. Gattungsgemäß weist die Gelenkschiene einen oberen oder proximalen Gelenkarm und einen damit teilweise überlappenden unteren oder distalen Gelenkarm auf. Die beiden Gelenkarme sind in mindestens einer Gelenkachse aneinander gleitend gegeneinander verschwenkbar. Der flache obere Gelenkarm und der flache untere Gelenkarm besitzen eine gemeinsame Gelenkachse, die zu der Fläche der Gelenkarme senkrecht steht. Im Bereich des Gelenkachse überlappen die beiden Gelenkarme und gleiten aneinander.

Erfindungsgemäß ist an dem oberen Gelenkarm ein so genannter Pelottenschlitten, der eine Pelotte trägt, vorhanden, der dort verschieblich in einer Schlittenschiene gelagert ist, wie in Anspruch 1 beschrieben. Der Pelottenschlitten ist offenbarungsgemäß gegen den Gelenkarm, der ihn trägt, linear, besonders in einer geraden Linie oder altematv auf einer Kreis- oder Kurvenbahn verlaufend, verschiebbar. Der Pelottenschlitten ist dazu offenbarungsgemäß mit dem anderen, insbesondere unteren oder distalen, Gelenkarm über ein Getriebe zwangsgeführt gekoppelt. Das Getriebe ist besonders ein Kurvengetriebe oder ein Koppelgetriebe; es dient generell der Umsetzung einer Drehbewegung, das heißt Verschwenkung, in eine drehwinkelabhängige Schubbewegung. Die aktiv geführte Schubbewegung ist durch Dimensionierung der Getriebeelemente vorgegeben, das heißt programmiert. Die Drehbewegung ergibt sich aus der Verschwenkung der beiden Gelenkarme gegeneinander; die Schubbewegung wird dabei an den Pelottenschlitten vermittelt, der dabei in der Schlittenschiene des oberen Gelenkarms gleitet.

Besonders ist der Pelottenschlitten dabei über das Getriebe mit dem unteren Gelenkarm derart gekoppelt, dass die Verschwenkung der beiden Gelenkarme, besonders bei Beugung oder Streckung des Körpergelenks, eine aktive Verschiebung des Pelottenschlittens an dem oberem Gelenkarm vermitteln kann. Offenbarungsgemäß wird dabei durch das Getriebe die Verschwenkung oder Drehbewegung der beiden Gelenkarme in eine Schubbewegung der Pelotte nach distal umgewandelt.

In der Ausführung als Hüftgelenksorthese übt die über dem Throchanter (Großer Rollhügel) liegende Pelotte einen abwärts (nach distal) gerichteten Impuls auf diesen Knochenvorsprung aus, welcher zu einer Dehnung und gleichsam Beweglichkeitsverbesserung und damit zur Schmerzlinderung führen (Krankengymnastisches Prinzip der "Extensions-behandlung").

Vorteilhafterweise ist die Pelotte derart mit dem Pelottenschlitten verbunden und an der Gelenkschiene orientiert, dass sie im angelegten Zustand der Gelenkorthese an einem Körperabschnitt nahe des Körpergelenks positioniert ist, der eine Schmerzlinderung bei der Gelenkbewegung bewirkt. Die Pelottenbewegung ist dabei vorzugsweise eine Auf- und Abbewegung und wird bei jeder Streckung oder Beugung des Körpergelenks aktiv ausgelöst. Dabei ist bevorzugt keine Beschränkung auf eine rein massierende Wirkung der Pelotte vorgesehen, da bei der Hüftgelenksorthese die aktiv bewegte Pelotte ja in der entspannten Schwungphase des jeweiligen Beines auch leicht die Extremität selbst nach unten zieht.

Es wurde überraschend gefunden, dass die aktiv bewegte Pelotte den empfundenen Schmerz bei der Gelenkbewegung, besonders bei arthrotischen, kontrakten, bewegungseingeschränkten Gelenken, spürbar vermindert. Auf diese Weise kann der Tragekomfort und die Compliance der Gelenkorthese bei dem Patienten und damit der Therapieerfolg deutlich verbessert werden. Die Gelenkorthese wird nicht mehr als störendes technisches Konstrukt empfunden, sondern als unmittelbar schmerzlindernd wirkendes Hilfsmittel akzeptiert.

Ein besonderer Aspekt der vorliegenden Erfindung besteht darin, dass die impulsgebende Pelotte aktiv durch die Verschwenkung der beiden Gelenksschienenarme zueinander zwangsgeführt wird.

In einer bevorzugten Ausgestaltung ist das verkoppelnde Getriebe ein Kurvengetriebe. Dies kann in an sich bekannter Weise aus einer Kurvenbahn und einem darauf ablaufenden Abtaster gebildet sein. In einer bevorzugten Ausgestaltung ist das Kurvengetriebe durch eine an oder in dem unteren Gelenkarm ausgebildete, insbesondere ebene Kurvenbahn und eine daran zwangsgeführte Rolle gebildet. In einer bevorzugten Variante dieses Kurvengetriebes ist die Kurvenbahn in dem unteren Gelenksarm als Nut ausgebildet. Darin läuft die Rolle zwangsgeführt nach Art einer Kulissensteuerung.

Die Rolle ist mit dem Pelottenschlitten des oberen Gelenkarms verkoppelt. Vorzugsweise ist dies eine starre Kopplung, wobei Pelottenschlitten und Koppelelement, besonders in Form einer Schubstange, einstückig ausgebildet sein können. In einer alternativen Ausgestaltung ist das Koppelelement zwischen Rolle und Pelottenschlitten mit dem Pelottenschlitten gelenkig verbunden.

Um die Auf- und Abbewegung der Pelotte an dem oberen Gelenkarm zu vermitteln, ist der Pelottenschlitten bevorzugt in einer im Wesentlichen radial zu der Gelenkachse der Gelenkschiene orientierten, insbesondere geraden, Schlittenschiene geführt. Diese ist an oder in dem oberen Gelenkarm ausgebildet, vorzugsweise in Form einer Nut. In einer Variante davon ist der Pelottenschlitten in einer Kurvenbahn geführt, beispielsweise in einem Kreisbogen oder einer Serpentine.

In einer weiteren alternativen Ausgestaltung ist vorgesehen, dass der Pelottenschlitten in dem oberen Gelenkarm auch oder ausschließlich drehbar gelagert ist und die durch das Getriebe vermittelte Schubbewegung in eine reine Rotationsbewegung des Pelottenschlittens oder in eine kombinierte Rotations- und Translationsbewegung des Pelottenschlittens - und folglich der Pelotte - an dem oberen Gelenkarm umgesetzt ist.

Um die gewünschte aktive Bewegung der Pelotte bei der Gelenkbewegung zu vermitteln, ist die Kurvenbahn spezifisch geformt. Dazu ist besonders vorgesehen, dass die Kurvenbahn mindestens einen schubaktiven Kurvenabschnitt neben zumindest einen schubneutralen Kurvenabschnitt aufweist. Der schubaktive Abschnitt vermittelt bei Verschwenkung der Gelenkarme, das heißt bei Drehbewegung, eine Schubbewegung der Rolle und folglich des Pelottenschlittens. Ein schubneutraler Abschnitt vermittelt im Gegensatz dazu bei Verschwenkung der Gelenkarme, das heißt bei Drehbewegung, keine Schubbewegung der Rolle und folglich des Pelottenschlittens, weil insbesondere die Kurvenbahn auf einem Kreisbogen mit konstantem Radius zu der Gelenkachse verläuft.

Bevorzugt ist vorgesehen, dass die Bewegung der Pelotte, insbesondere dann oder alternativ genau und ausschließlich dann ausgelöst wird, wenn das Körpergelenk entlastet ist. Dies ist beim Hüft- oder Kniegelenk besonders in der Bewegungsphase des Durchschwingens des Beines beim Schreiten der Fall, das heißt bei vollständiger Streckung des Gelenks, entsprechend 0° Gelenkwinkel zwischen den beiden Gelenkarmen. Dazu ist ein einer bevorzugten Ausführung vorgesehen, dass die Kurvenbahn zumindest einen, oder alternativ genau einen, schubaktiven Kurvenabschnitt aufweist, und zwar vor allem oder alternativ ausschließlich an derjenigen Position der Kurvenbahn, wo durch eine Schubauslenkung der Rolle und damit Verschiebung des Pelottenschlittens im Bereich der vollen Extension des Orthesengelenks, das heißt bei einem Gelenkwinkel von 180° , vermitteln kann.

In einer alternativen Variante sind auf der Kurvenbahn mehrere schubaktive Kurvenabschnitte angeordnet, vorzugsweise im Bereich der vollständigen Streckung des Gelenks, entsprechend 0° Gelenkwinkel zwischen den beiden Gelenkarmen, sowie zusätzlich im Bereich der beiden Verschwenkungsendpunkte, das heißt bei vollständiger Beugung des Gelenks. In bevorzugter Ausgestaltung davon weist die Kurvenbahn drei schubaktive Kurvenabschnitte auf. In einer weiteren alternativen Variante ist die Kurvenbahn serpentinenförmig ausgestaltet, sodass entlang des gesamten Verschwenkbereichs eine aktive Bewegung der Pelotte vermittelt wird.

In anderen Ausgestaltungen der Offenbarung ist das Getriebe ein Koppelgetriebe und verwirklicht die Umsetzung der Verschwenkung, das heißt Drehbewegung, der beiden Gelenkarme der Gelenkschiene in die im Wesentlichen lineare Schubbewegung der Pelotte vermittelst Koppelstangen zwischen unterem Gelenkarm und Pelottenschlitten. In einer anderen Variante der Offenbarung ist zu dieser offenbarungsgemäßen Bewegungsumsetzung eine Kombination aus Rollen und Seilen vorgesehen, wobei die Seile mit dem unteren Gelenkarm verkoppelt sind und über Rollen, welche in bevorzugter Ausgestaltung an dem oberen Gelenkarm angeordnet sind, mit dem Pelottenschlitten verbunden sind.

In einer besonderen Ausgestaltung der Schiene trägt der insbesondere obere Gelenkarm in der Nähe zu der mindestens einen aktiv beweglichen Pelotte mindestens eine in Bezug auf diese Gelenkschiene feststehende Pelotte. Aktiv bewegliche und feststehende Pelotte(n) haben im Zusammenspiel eine verbesserte Wirkung. In einer ersten Variante sind feststehende Pelotten seitlich zu der aktiv bewegten Pelotte angeordnet. In einer dazu alternativen oder zusätzlichen Variante ist mindestens eine feststehende Pelotte oberhalb zu der aktiv bewegten Pelotte angeordnet. Ist die Gelenkschiene in einer Hüftorthese eingesetzt ist bevorzugt vorgesehen, dass die mindestens eine feststehende Pelotte oberhalb des Hüftgelenks, bevorzugt am großen Rollhügel (*Trochanter major femoris*) abgeordnet; die aktiv bewegliche Pelotte ist dabei bevorzugt im Bereich der glutealen Muskulatur, besonders *Musculus gluteus medius* und/oder *Musculus gluteus minimus*, angeordnet.

Gegenstand der Offenbarung ist auch eine Gelenkorthese, vorzugsweise eine Hüftgelenksorthese oder eine Kniegelenksorthese, welche die offenbarungsgemäße Orthesengelenkschiene, wie hierin beschrieben, enthält. Eine solche Gelenkorthese besteht zumindest aus einer oberen Fassung des proximalen Körperteils und einer unteren Fassung des distalen Körperteils. Beide Fassungen sind über mindestens eine, im Falle einer Kniegelenksorthese vorzugsweise zwei offenbarungsgemäße Gelenkschienen verbunden.

Ein weiterer Aspekt der Offenbarung ist Verwendung der offenbarungsgemäßen Orthesengelenkschiene in der Prophylaxe und Therapie, spezifisch von Arthrosen, insbesondere bei Patienten, die unter Arthrose leiden. Ein bevorzugter Verwendungszweck ist dabei die Verbesserung des Tragekomforts und/oder der Compliance einer Gelenkorthese, spezifisch im Rahmen der vorgenannten Prophylaxe oder Therapie.

Die Offenbarung wird an den nachfolgenden Beispielen näher erläutert, ohne dass die beschränkend zu verstehen wären:

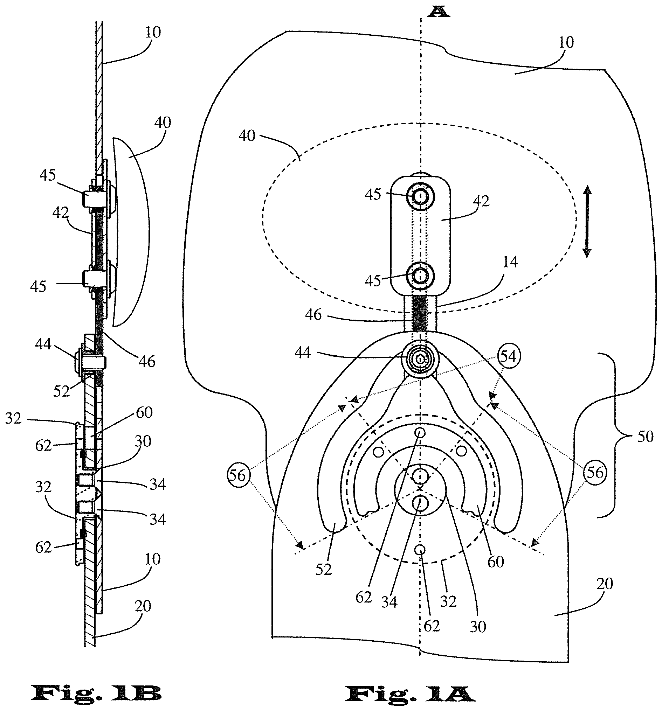

Figur 1A zeigt eine schematische Darstellung in Draufsicht einer Ausführung der erfindungsgemäßen Gelenkschiene. An einem oberen Gelenkarm (10), der besonders als Oberschale ausgebildet ist, ist an dessen Basis ein Axialgelenk oder Gelenkachse (30) vorgesehen, die diesen oberen Gelenkarm (10) mit einem darauf überlappend liegenden unteren Gelenkarm (20) verbindet, sodass der untere Gelenkarm (20) in der Gelenkachse (30) an dem oberen Gelenkarm (10) gleitend verschwenkbar ist. Dabei wird der untere Gelenkarm (20) in zwischen dem oberen Gelenkarm (10) und einem damit über Bolzen (34) geschulterten Gelenkteller (32) geführt.
   Erfindungsgemäß ist an oder in dem oberen Gelenkarm (10) eine Schlittenschiene (14) ausgebildet, worin ein Pelottenschlitten (42) praktisch spielfrei gleiten kann. Der Pelottenschlitten (42) ist bevorzugt aus zwei, beiderseits des flachen Gelenkarms (10) angeordneten Platten gebildet, die über Bolzen (45) miteinander verbunden sind. Der Pelottenschlitten (42) trägt mindestens eine, vorzugsweise gepolsterte, Pelotte (40). Der Pelottenschlitten (42) ist in der dargestellten Ausführung über eine starre Schubstange (46), die in der Ausnehmung der Schlittenschiene (14) parallel zu dieser verläuft, mit einer Rolle (44) starr verbunden. Die Rolle (44) dient als Abtaster einer Kurvenbahn (52), die als flache Kulissennut in dem schwenkbaren unteren Gelenkarm (20) ausgebildet ist. Die Rolle (44) wird in der Kulisse in Bezug auf die Gleitrichtung des Pelottenschlittens (42) in der Schlittenschiene (14) praktisch spielfrei zwangsgeführt. Um die Gelenkachse (30) schwenkbare Kurvenbahn (52) und geführte Rolle (44) bilden ein erfindungsgemäßes Kurvengetriebe (50), das eine lineare Schub-/Gleitbewegung des Pelottenschlittens (42) in Abhängigkeit von dem Verschwenkwinkel der beiden Gelenkschenkel (10,20) in dem Axialgelenk (30) vermittelt.
   Die Kurvenbahn (52) ist in der dargestellten Ausführung so ausgebildet, dass schubneutrale Kurvenabschnitte (56) neben einem, hier mittig angeordneten, schubaktiven Kurvenabschnitt (54) vorhanden sind. Die schubneutralen Kurvenabschnitte (56) sind dadurch gekennzeichnet, dass sie zu der Gelenkachse (30) einen konstanten radialen Abstand besitzen, sodass dabei keine Hubbewegung an der dort laufenden Rolle (44) vermittelt wird. Im Gegensatz dazu ist der schubaktive Kurvenabschnitt 56 so ausgestaltet, dass, sobald die Rolle (44) bei der Verschwenkung in diesen Abschnitt läuft, eine lineare Verschiebung der geführten Rolle (44) und damit ein Auf- oder Abgleiten des Pelottenschlittens (42) in der Schlittenschiene (14) vermittelt wird. In der dargestellten Ausgestaltung findet diese Hubbewegung (Pfeil) im Bereich der vollständigen Streckung des Gelenks, das heißt bei einem Gelenkwinkel von etwa 0° statt.
   Weiter ist die Gelenkschiene optional mit Mitteln zur Verschwenkungslimitierung versehen. Dazu ist in dem unteren Gelenkarm (20) eine kreisbogenförmige Ausnehmung (60) innerhalb des Durchmessers des verdrehfest an dem oberen Gelenkarm (10) gesicherten Gelenktellers (32) ausgebildet, und in dem Gelenkteller (32) sind eines oder mehrere Löcher (62) zur wahlweisen Aufnahme von Blockierstiften (nicht gezeigt) vorhanden. Es ist vorgesehen, dass diese Blockierstifte in der darunterliegenden Ausnehmung (60) laufen und ermöglichen, je nach ihrer Positionierung, eine Limitierung des Verschwenkwinkels der Gelenkarme (10, 20) zu einander. Die Positionierung der Löcher (62) ist beispielhaft gezeigt. Andere Positionen entlang des Kreisbogens (60) sind möglich.
Figur 1B zeigt die Ausführung nach Figur 1 in Längsschnittansicht in Schnittlinie A.

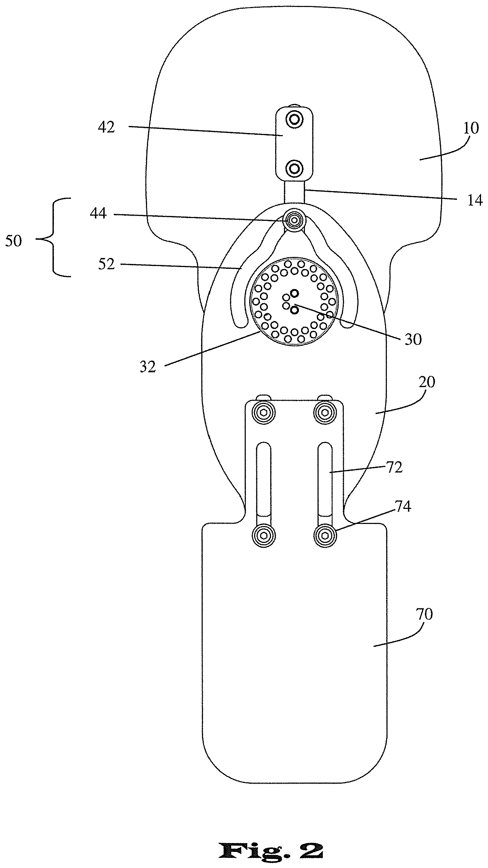

Figur 2 zeigt eine schematische Darstellung in Draufsicht einer Variante der Ausführung nach Figur 1A und 1B, spezifisch ausgebildet als Gelenkschiene für eine Hüftorthese. Der untere Gelenkarm (20) trägt dabei eine über Langlöcher (72) längenverstellbare Schiene (70), die an dem Gelenkarm (20) über Bolzen (74) fixierbar ist. Die an dem Pelottenschlitten (42) auf- und abrüstbare Pelotte (40) rückseitig angebracht und ist nicht dargestellt. Der Gelenkteller (32) weist eine Vielzahl von Bohrungen (62) auf zur feindosierbaren Limitierung der Verschwenkung mittels dort einsetzbarer Blockierstifte (nicht gezeigt).

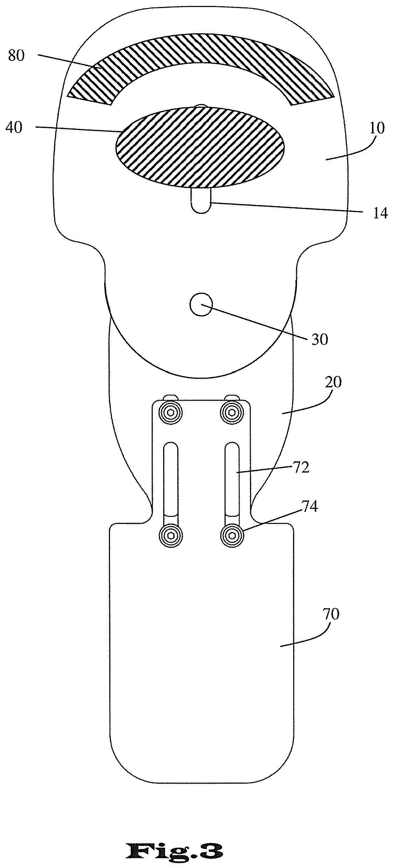

Figur 3 zeigt eine schematische Darstellung in Draufsicht der Rückseite der Ausführung nach Figuren 1A und 1B. Die aktiv bewegliche Pelotte (40) ist dort angeordnet und zeigt im angelegten Zustand der Orthese Richtung Gelenk. In der hier dargestellten Variante ist optional zusätzlich mindestens eine, an dem oberen Gelenkarm (10) ortsfest fixierte feststehende Pelotte (80) vorgesehen.

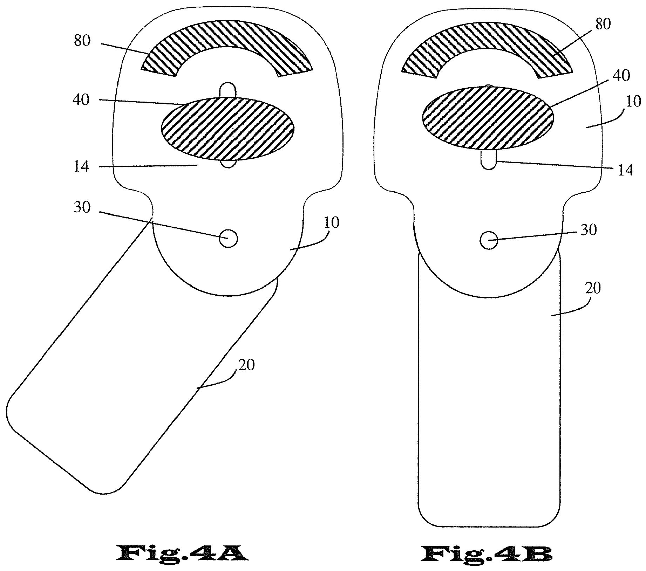

Figuren 4A und 4B zeigen, jeweils in schematischer Darstellung in Draufsicht der Rückseite eine Variante der Ausführung nach Figur 3, das Funktionsprinzip der erfindungsgemäßen Gelenkschiene. Die aktiv bewegliche Pelotte (40) ist über den Pelottenschlitten (nicht gezeigt) in Schiene (14) geführt und in ihrer Position abhängig von dem Winkel der Verschwenkung des unteren Gelenkarms (20) zu dem oberen Gelenkarm (10) in der Gelenkachse (30). Diese Winkelabhängigkeit wird durch das erfindungsgemäße Getriebe (nicht gezeigt) vermittelt. In Figur 4A ist die Situation bei Beugung des Gelenks dargestellt. In Figur 4B ist die Situation bei Streckung des Gelenks (Gelenkwinkel 0°) dargestellt: die aktiv bewegte Pelotte (40) ist dabei aktiv gegenüber einer Ruhelage in Richtung der feststehenden Pelotte (80) verschoben.

## Patentansprüche

1. Orthesengelenkschiene mit flachem oberem Gelenkarm (10) und in einer dazu senkrechten Gelenkachse (30) verschwenkbar gelagertem, im Bereich des Gelenkachse überlappendem, flachem unterem Gelenkarm (20), **dadurch gekennzeichnet, dass**
an oder in dem oberen Gelenkarm (10) ein Pelottenschlitten (42) mit Pelotte (40) in einer Schlittenschiene (14) verschieblich gelagert ist und der Pelottenschlitten (42) mit dem unterem Gelenkarm (20) über ein Getriebe (50), ausgewählt aus Kurvengetriebe und Koppelgetriebe, zwangsgeführt gekoppelt ist.

2. Orthesengelenkschiene nach Anspruch 1, wobei der Pelottenschlitten (42) mit dem unterem Gelenkarm (20) über das Getriebe (50) derart gekoppelt ist, dass die Verschwenkung der beiden Gelenkarme (10, 20) zu einander eine lineare Verschiebung des Pelottenschlittens (42) an dem oberen Gelenkarm (10) vermitteln kann.

3. Orthesengelenkschiene nach Anspruch 1 oder 2, wobei das Getriebe (50) ein Kurvengetriebe ist, das aus einer an oder in dem unterem Gelenkarm (20) ausgebildeten Kurvenbahn (52) und einer daran zwangsgeführten Rolle (44), die mit dem Pelottenschlitten (42) des oberen Gelenkarms (10) verkoppelt ist, gebildet ist.

4. Orthesengelenkschiene nach Anspruch 3, wobei die Kurvenbahn (52) als Nut in dem unterem Gelenkarm (20) ausgebildet ist

5. Orthesengelenkschiene nach einem der Ansprüche 3 oder 4, wobei die Kurvenbahn (52) mindestens einen schubaktiven Kurvenabschnitt (54) neben zumindest einen schubneutralen Kurvenabschnitt (56) ausweist.

6. Orthesengelenkschiene nach Anspruch 5, wobei die Kurvenbahn (52) einen schubaktiven Kurvenabschnitt (54) aufweist, der eine Schubauslenkung der Rolle (44) und die Verschiebung des Pelottenschlittens (42) im Bereich der vollen Extension des Orthesengelenks, das heißt Schwenkwinkel = 0° , vermitteln kann.

7. Orthesengelenkschiene nach einem der vorstehenden Ansprüche, wobei der Pelottenschlitten (42) in der Schlittenschiene (14) radial zu der Gelenkachse (30) geführt ist.

8. Orthesengelenkschiene, zusätzlich enthaltend an dem oberen Gelenkarm (10) mindestens eine weitere Pelotte (80), die feststehend ist.

9. Gelenkorthese, enthaltend die Orthesengelenkschiene nach einem der vorstehenden Ansprüche.

10. Gelenkorthese nach Anspruch 9, ausgewählt aus Hüftgelenksorthese und Kniegelenksorthese

## Claims

1. An orthotic joint splint having a flat upper articulated arm (10) and a flat lower articulated arm (20), which is mounted pivotably in an articulation axis (30) perpendicular to said upper articulated arm and which overlaps said arm in the region of the articulation axis, **characterized in that**
on or in the upper articulated arm (10) a pad carriage (42) with a pad (40) is displaceably mounted in a carriage track (14) and the pad carriage (42) is coupled in a positively guided manner to the lower articulated arm (20) via a motion transmitting mechanism (50), selected from cam mechanisms and mechanical linkages.

2. The orthotic joint splint according to claim 1, wherein the pad carriage (42) is coupled to the lower articulated arm (20) via the motion transmitting mechanism (50) such that the pivoting of the two articulated arms (10, 20) relative to one another can convey a linear displacement of the pad carriage (42) on the upper articulated arm (10).

3. The orthotic joint splint according to claim 1 or 2, wherein the motion transmitting mechanism (50) is a cam mechanism composed of a cam track (52) formed on or in the lower articulated arm (20) and a roller (44) that is positively guided therein and is coupled to the pad carriage (42) of the upper articulated arm (10).

4. The orthotic joint splint according to claim 3, wherein the cam track (52) is configured as a groove in the lower articulated arm (20).

5. The orthotic joint splint according to either of claims 3 and 4, wherein the cam track (52) has at least one cam section which is active with regard to thrust (54) and at least one cam section which is neutral with regard to thrust (56).

6. The orthotic joint splint according to claim 5, wherein the cam track (52) has a cam section which is active with regard to thrust (54) that can convey a thrust deflection of the roller (44) and the displacement of the pad carriage (42) in the region of the full extension of the orthotic joint, i.e., at pivot angle = 0°.

7. The orthotic joint splint according to any one of the preceding claims, wherein the pad carriage (42) is guided in the carriage track (14) radially to the articulation axis (30).

8. The orthotic joint splint, additionally comprising at least one additional, fixed pad (80) on the upper articulated arm (10).

9. A joint orthosis comprising the orthotic joint splint according to any one of the preceding claims.

10. The joint orthosis according to claim 9, selected from hip joint orthosis and knee joint orthosis.

## Revendications

1. Attelle articulée d'orthèse avec bras articulé supérieur plat (10) et bras articulé inférieur plat (20) pouvant pivoter dans un axe d'articulation (30) perpendiculaire à celui-ci et en chevauchement dans la zone de l'axe d'articulation, **caractérisée en ce que,**
sur ou dans le bras articulé supérieur (10), une glissière de pelotte (42) avec pelotte (40) est montée de manière à pouvoir coulisser dans une attelle à glissière (14) et **en ce que** la glissière de pelotte (42) est couplée par guidage forcé avec le bras articulé inférieur (20) par un engrenage (50) choisis entre la commande à came et l'engrenage de couplage.

2. Attelle articulée d'orthèse selon la revendication 1, dans laquelle la glissière de pelotte (42) étant couplée avec le bras articulé inférieur (20) par l'engrenage (50) de telle sorte que le pivotement des deux bras articulés (10, 20) l'un par rapport à l'autre puisse transmettre un déplacement linéaire de la glissière de pelotte (42) sur le bras articulé supérieur (10).

3. Attelle articulée d'orthèse selon revendication 1 ou 2, dans laquelle l'engrenage (50) étant une commande à came qui est constituée d'une voie courbée (52) formée sur ou dans le bras articulé inférieur (20) et d'un rouleau (44) à guidage forcé qui est couplé avec la glissière de pelotte (42) du bras articulé supérieur (10).

4. Attelle articulée d'orthèse selon la revendication 3, dans laquelle la voie courbée (52) étant conçue comme rainure dans le bras articulé inférieur (20).

5. Attelle articulée d'orthèse selon l'une quelconque des revendications 3 ou 4, dans laquelle la voie courbée (52) présentant au moins une section de courbe active vis-à-vis de la poussée (54) à côté d'au moins une section de courbe neutre vis-à-vis de la poussée (56).

6. Attelle articulée d'orthèse selon la revendication 5, dans laquelle la voie courbée (52) présentant une section de courbe active vis-à-vis de la poussée (54), pouvant transmettre une déviation de poussée du rouleau (44) et le déplacement de la glissière de pelotte (42) dans la zone de l'extension totale de l'articulation d'orthèse, c'est-à-dire angle de pivotement = 0°.

7. Attelle articulée d'orthèse selon l'une quelconque des revendications précédentes, la glissière de pelotte (42) étant guidée dans l'attelle à glissière (14) radialement par rapport à l'axe d'articulation (30).

8. Attelle articulée d'orthèse, contenant en plus, sur le bras articulé supérieur (10), au moins une autre pelotte (80) qui est fixée.

9. Orthèse d'articulation contenant l'attelle articulée d'orthèse selon l'une des revendications précédentes.

10. Orthèse d'articulation selon la revendication 9, choisi entre l'orthèse de l'articulation de la hanche et l'orthèse de l'articulation du genou.
